# EUROPEAN PATENT APPLICATION

(11) **EP 4 810 947 A1**
(43) Date of publication of application: **23.09.2026**
(21) Application number: 24887707.8
(22) Date of filing: 14.10.2024
(51) Int. Cl.: G01N 23/046, A61B 6/03, A61B 6/02

(54) **CT SCANNING SYSTEM AND METHOD**

(30) Priority: 09.11.2023 CN 202311490575
(71) Applicant: Tsinghua University, Haidian District, Beijing 100084 (CN); NUCTECH COMPANY LIMITED, Beijing 100084 (CN)
(72) Inventor: CHEN, Zhiqiang, Beijing 100084 (CN); ZHANG, Li, Beijing 100084 (CN); SHEN, Le, Beijing 100084 (CN); SUN, Yunda, Beijing 100084 (CN); HUANG, Qingping, Beijing 100084 (CN)
(74) Representative: Gunzelmann, Rainer
(86) International application number: PCT/CN2024/124714
(87) International publication number: WO 2025/098094

(57) **Abstract**

A CT scanning system is provided, including: a conveying device (3), configured to move a scanned object (30) in a scanning channel (31) in a conveying direction; a distributed ray source (1) including m target points (10), where the m target points (10) are configured to be activated in a predetermined order to emit ray beams, m is a positive integer greater than or equal to 2, the ray beams emitted by at least one of the m target points (10) is modulated into a first ray beam having a first energy, and the ray beams emitted by at least another of the m target points (10) is modulated into a second ray beam having a second energy, where the first energy is lower than the second energy; a detector array (2) configured to generate first projection data in response to the first ray beam passing through the scanned object (30), and generate second projection data in response to the second ray beam passing through the scanned object (30); and an image reconstruction device (4) configured to generate a computed tomography image of the scanned object (30) according to the first projection data and the second projection data. The distributed ray source (1) is configured to rotate around a first axis when the scanned object (30) is scanned, and the first axis is parallel to the conveying direction.

## Description

This application claims the priority of Chinese Patent Application No. 202311490575.1 filed on November 09, 2023 in the China National Intellectual Property Administration, the content of which is incorporated herein by reference in entirety.

### TECHNICAL FIELD

The present disclosure relates to a field of radiation scanning, and in particular to a CT scanning system and a CT scanning method.

### BACKGROUND

Computed Tomography (CT) scanning technology is widely used in medical inspection, security inspection, industrial inspection, and the like. For example, CT scanning systems used in the security inspection field may detect items such as baggage, parcels, and the like.

In CT scanning techniques, structural information inside an object is obtained using different degrees of absorption of X-rays through the object. Generally, a CT scanning system may include a ray source, a detector, and a computer system. For example, an X-ray source emits X-rays to pass through an object to be detected, a detector receives the X-rays passing through the object to be detected and converts the X-rays into electrical signals, the electrical signals are amplified and digitized and transmitted to a computer system, and the computer system processes the received data using an image reconstruction algorithm to generate a two-dimensional or three-dimensional image. CT scanning technology combines X-ray imaging and computer image reconstruction techniques to provide high resolution, three-dimensional image data that may help security personnel accurately detect potentially dangerous items or other security threats.

With the development of the technology, the spiral CT scanning technology is more and more widely applied to the fields of medical examination, security inspection, industrial detection, and the like. In a spiral CT scanning system, tomography image data of an object to be detected is acquired by means of continuous rotational scanning. Compared with the conventional CT scanning technology, the spiral CT scanning technology has the advantages of high scanning speed, high space-time resolution and the like. However, how to further increase the scanning speed of the spiral CT scanning system is one of the important issues that researchers are always concerned about.

The above information disclosed in this section is only for understanding of the background of the disclosed concept of the present disclosure, and thus, the above information may contain information that does not constitute related art.

### SUMMARY

The present disclosure provides a CT scanning system and a CT scanning method.

According to a first aspect of the present disclosure, a CT scanning system is provided, including:
a conveying device, configured to move a scanned object in a scanning channel in a predetermined conveying direction;
a distributed ray source, wherein the distributed ray source includes m target points, the m target points are configured to be activated in a predetermined order to emit ray beams, m is a positive integer greater than or equal to 2, the ray beams emitted by at least one of the m target points is modulated into a first ray beam having a first energy, and the ray beams emitted by at least another of the m target points is modulated into a second ray beam having a second energy, wherein the first energy is lower than the second energy;
a detector array, wherein the detector array is configured to detect a ray emitted from the distributed ray source and passing through the scanned object, and to generate projection data according to the detected ray, wherein the detector array is configured to generate first projection data in response to the first ray beam passing through the scanned object, and generate second projection data in response to the second ray beam passing through the scanned object, and
an image reconstruction device, wherein the image reconstruction device is configured to generate a computed tomography image of the scanned object according to the first projection data and the second projection data,
wherein the distributed ray source is configured to rotate around a first axis when the scanned object is scanned, and the first axis is parallel to the conveying direction.

According to some exemplary embodiments, the system further includes a voltage supply device, the voltage supply device is configured to respectively provide voltage to the m target points of the distributed ray source; and the voltage supply device is configured to supply a first voltage to at least one of the m target points, and supply a second voltage to at least another of the m target points, wherein the first voltage is lower than the second voltage.

According to some exemplary embodiments, the system further includes a voltage supply device, the voltage supply device is configured to respectively provide voltage to the m target points of the distributed ray source; and the voltage supply device is configured to supply a first voltage to at least one of the m target points, and supply a second voltage to at least another of the m target points, wherein the first voltage is substantially equal to the second voltage.

According to some exemplary embodiments, the system further includes at least one ray filter, the at least one ray filter is arranged on a beam path of at least one of the m target points, and is configured to modulate the ray beams emitted by at least one of the m target points into the first ray beam having the first energy.

According to some exemplary embodiments, the system further includes a controller, the controller is configured to control at least one aspect of the distributed ray source, including: activation moment of m target points, duration of ray beams emitted by m target points, intensity of ray beams emitted by m target points, and energy of ray beams emitted by m target points.

According to some exemplary embodiments, in the distributed ray source, the m target points are arranged at intervals in a first arrangement direction, and the first arrangement direction is parallel to the first axis.

According to some exemplary embodiments, the distributed ray source includes an X-ray generating tube having a plurality of target points, or the distributed ray source includes a plurality of X-ray generating tubes having a single target point.

According to some exemplary embodiments, the detector array includes a single row of detectors.

According to some exemplary embodiments, the detector array includes n rows of detectors, and n is a positive integer greater than or equal to 2.

According to some exemplary embodiments, the detector array includes an area array detector.

According to some exemplary embodiments, the detector array includes n1 rows of detectors, n1 is a positive integer greater than or equal to 1, and m is greater than n1.

According to some exemplary embodiments, the m target points are arranged at intervals in a first arrangement direction, the first arrangement direction is parallel to the first axis; and the n rows of detectors are arranged at intervals in the first arrangement direction.

According to some exemplary embodiments, in the distributed ray source, at least two of the m target points are offset in a tangent direction, and the tangent direction is a direction extending along a tangent line tangent to a rotation direction of the distributed ray source.

According to some exemplary embodiments, the CT scanning system further includes a post collimator, and the post collimator is located on a side of the detector array facing the distributed ray source.

According to some exemplary embodiments, each row of detectors includes a plurality of detector modules, and in a plane perpendicular to the first axis, a plurality of detector modules of at least one row of detectors are arranged continuously along an arc line; and in a plane perpendicular to the first axis, at least one target point is offset from an arc center of the arc line.

According to a second aspect of the present disclosure, a CT scanning method is provided, including:
driving, by a conveying device, a scanned object to move in a scanning channel in a predetermined conveying direction;
activating m target points of a distributed ray source in a predetermined order to emit ray beams, thereby forming a scanning region, wherein m is a positive integer greater than or equal to 2, the ray beams emitted by at least one of the m target points is modulated into a first ray beam having a first energy, and the ray beams emitted by at least another of the m target points is modulated into a second ray beam having a second energy, wherein the first energy is lower than the second energy;
driving the scanned object to pass through the scanning region;
in a process of the scanned object passing through the scanning region, detecting, by a detector array, a ray emitted from the distributed ray source and passing through the scanned object, and generating projection data according to the detected ray, wherein first projection data is generated in response to the first ray beam passing through the scanned object; and second projection data is generated in response to the second ray beam passing through the scanned object; and
generating a computed tomography image of the scanned object according to the first projection data and the second projection data,
wherein the distributed ray source is configured to rotate around a first axis when the scanned object is scanned, and the first axis is parallel to the conveying direction.

### BRIEF DESCRIPTION OF THE DRAWINGS

In order to better understand the present disclosure, the present disclosure will be described in detail according to the following drawings:
FIG. 1 is a schematic structural view of a CT scanning system according to some exemplary embodiments of the present disclosure;
FIG. 2A is a scanning schematic diagram of a conventional CT scan;
FIG. 2B is a scanning schematic diagram of a CT scanning using a distributed ray source according to an embodiment of the present disclosure;
FIG. 3A is a structural schematic diagram of a distributed ray source, according to some exemplary embodiments of the present disclosure;
FIG. 3B is a schematic diagram of the distributed ray source of FIG. 3A viewed from another perspective;
FIG. 3C schematically shows a waveform diagram of a current used to control a beam output of a distributed ray source;
FIG. 4 is a schematic structural diagram of yet another distributed ray source according to some exemplary embodiments of the present disclosure;
FIG. 5A is a schematic structural diagram of a combination of a source and a detector of a CT scanning system in which a distributed ray source and a single row of detectors are schematically illustrated according to an embodiment of the present disclosure; FIG. 5B is a schematic diagram of a combination of a source and a detector of a conventional CT scanning system in which a single-target ray source and a plurality of rows of detectors are schematically illustrated;
FIG. 5C is a schematic structural diagram of a combination of a source and a detector of a CT scanning system in which a distributed ray source and a plurality of rows of detectors are schematically illustrated according to an embodiment of the present disclosure; FIG. 5D is a schematic diagram of a combination of a source and a detector of a conventional CT scanning system in which a single-target ray source and further rows of detectors are schematically illustrated;
FIG. 5E is a schematic structural diagram of a combination of a source and a detector of a CT scanning system in which a distributed ray source and an area array detector are schematically illustrated according to an embodiment of the present disclosure; FIG. 5F is a schematic diagram of a combination of a source and a detector of a conventional CT scanning system in which a single-target ray source and an area array detector are schematically illustrated;
FIGS. 6A to 6D schematically illustrate some arrangements of a source and a detector respectively according to some exemplary embodiments of the present disclosure;
FIG. 7A to 7C respectively schematically illustrate an arrangement of a plurality of target points of a distributed ray source in a CT scanning system according to some exemplary embodiments of the present disclosure, where FIG. 7A is a perspective view of the distributed ray source, FIG. 7B is a schematic view of a source and a detector in a rotation state, and FIG. 7C is a schematic view of a source and a detector in another rotation state;
FIG. 8A schematically illustrates an arrangement of a source and a detector in a conventional CT scanning system, FIG. 8B schematically illustrates an exemplary arrangement of a source and a detector in the CT scanning system according to an embodiment of the present disclosure, and FIG. 8C schematically illustrates an installation structure of a source and a detector in the CT scanning system according to an embodiment of the present disclosure;
FIG. 9 schematically illustrates a post collimator included in a CT scanning system according to some exemplary embodiments of the present disclosure;
FIG. 10A schematically illustrates a scanning range of a conventional CT scanning system, and FIG. 10B schematically illustrates a scanning range of a CT scanning system according to some exemplary embodiments of the present disclosure;
FIG. 11 is a flow chart of a CT scanning method according to some exemplary embodiments of the present disclosure;
FIG. 12A and 12B schematically illustrate a comparison of reconstruction results of the CT scanning system according to an embodiment of the present disclosure and a conventional spiral CT system under the same scanning parameters; and
FIG. 13 schematically shows a block diagram of an imaging device of a CT scanning system according to an embodiment of the present disclosure.

### DETAILED DESCRIPTION OF EMBODIMENTS

Specific embodiments of the present disclosure will be described in detail below. It should be noted that the embodiments described here are only for illustration and are not intended to limit the present disclosure. In the following description, a large number of specific details are set forth in order to provide a thorough understanding of the present disclosure. However, it is obvious to those of ordinary skill in the art that these specific details do not have to be adopted to implement the present disclosure. In other examples, in order to avoid confusing the present disclosure, well-known structures, materials or methods are not described in detail.

Throughout the specification, references to "one embodiment", "an embodiment", "an example" or "one example" mean that the specific features, structures or characteristics described in conjunction with the embodiment or example are included in at least one embodiment of the present disclosure. Therefore, the phrases "in one embodiment", "in an embodiment", "an example" or "one example" appearing in various places throughout the specification do not necessarily refer to the same embodiment or example. In addition, specific features, structures or characteristics may be combined in one or more embodiments or examples in any appropriate combination and/or sub-combination. In addition, those of ordinary skill in the art should understand that the term "and/or" used herein includes any and all combinations of one or more related listed items.

The terms used herein are only for the purpose of describing specific embodiments and are not intended to limit the present disclosure. The terms "include", "comprise", and the like, used herein indicate the existence of features, steps, operations and/or components, but do not exclude the existence or addition of one or more other features, steps, operations or components.

All terms (including technical and scientific terms) used herein have the meanings commonly understood by those skilled in the art unless otherwise defined. It should be noted that the terms used herein should be interpreted as having a meaning consistent with the context of this specification and should not be interpreted in an idealized or overly rigid manner.

Because the spiral CT scanning technology has the advantages of fast scanning speed, high spatial and temporal resolution, and the like, compared with the conventional CT scanning technology, the spiral CT scanning technology is more and more widely applied to the fields of medical examination, security inspection, industrial detection, and the like. In a spiral CT scanning system, tomography image data of an object to be detected is acquired by means of continuous rotational scanning.

For example, taking a spiral CT scanning system applied to a security inspection scene as an example, the spiral CT scanning system includes a ray source, a detector, a scanning channel, a control system and a computer system. The ray source and the detector are mounted on a gantry by a support member, and may rotate around a scanned object when the scanned object passes through the scanning channel. The control system is used to control the scanning parameters and the image acquisition process, such as scanning speed, dose, and the like. The computer system is used for image reconstruction, image processing and analysis. The basic principle of the spiral CT scanning technique is to acquire a tomography image of a scanned object by continuous rotational scanning and image reconstruction. Specifically, the scanned object is placed in a scanning channel, such as a luggage on a luggage conveyer. The X-ray source and detector are rotated to perform a continuous rotational scanning around the scanned object. The X-rays pass through the scanned object, are received by a detector, and are converted into electrical signals. The electrical signal is amplified and digitized before being transmitted to a computer system. The computer system processes the received data using a reconstruction algorithm to generate a reconstructed image. The spiral CT scanning systems may utilize continuous rotational scanning and image reconstruction techniques to provide high resolution tomography images.

Increasing the scanning speed of spiral CT scanning systems is one of the issues that those skilled in the relevant art continuously pay attention to. The inventor has found that, in recent years, the improvement of the spiral CT scanning speed mainly starts from two aspects, namely, the rotation speed of the slip ring is improved, and the number of rows of the detectors is increased.

However, for the spiral CT scanning device applied to the medical examination field, the slip ring rotation speed and the detector array number of the spiral CT scanning device have been increased to the limit level, and it becomes difficult to increase the scanning speed by means of further increasing the slip ring rotation speed or increasing the detector array number.

For the spiral CT scanning device applied to the security inspection field, because the size of a scanning channel is large, and the rotating speed of a slip ring of the spiral CT scanning device is restricted by the mechanical property of an imaging part, the scanning speed is difficult to improve by means of improving the rotating speed of the slip ring; in the CT scanning device, the detectors belong to components with high cost, and increasing the number of detector rows will significantly increase the number of detectors, and accordingly, the cost of the whole CT scanning device will also significantly increase.

The inventor further researches and discovers that in a spiral CT scanning device, the increase of the number of detector rows causes the problems of cone angle artifacts, the increase of the spatial solid angle of a ray bundle, the increase of the structural complexity of a post collimator and the like. For example, increasing the number of detector rows results in an increase in the spatial solid angle of the ray beam, which increases the scattering fraction and seriously affects the image quality and numerical accuracy; furthermore, as the spatial solid angle of the ray beam increases, the level of radiation protection needs to be increased, which results in increased device weight and scanning channel length.

The inventor has further studied and found that in the field of security inspection, a non-rotational static multi-source CT scanning technique may be adopted instead of the spiral CT scanning technique. In the static multi-source CT scanning technique, a plurality of ray sources are arranged around a scanning channel according to a certain geometric arrangement, and data similar to spiral scanning is generated by way of alternate beam exposure for image reconstruction. Because the frequency of the alternating beams from the plurality of ray sources may be fast, a higher scanning speed may be generated than the scanning speed of the slip ring rotation.

However, in a CT scanning device using a static multi-source CT scanning technique, since the detector needs to receive the ray emitted from the source at a different position, the post collimator may not be used to suppress the scattered signal. Moreover, the incident angles of the ray source at different positions and different target points on the detector are completely different, which results in different energy spectrums of the projection data at each angle, and is not beneficial to energy spectrum correction and dual-energy and multi-energy reconstruction.

In view of this, the embodiment of the present disclosure provides a CT scanning system, and the system includes: a conveying device, configured to move a scanned object in a scanning channel in a predetermined conveying direction; a distributed ray source, where the distributed ray source includes m target points, the m target points are configured to be activated in a predetermined order to emit ray beams, m is a positive integer greater than or equal to 2, the ray beams emitted by at least one of the m target points is modulated into a first ray beam having a first energy, and the ray beams emitted by at least another of the m target points is modulated into a second ray beam having a second energy, where the first energy is lower than the second energy; a detector array, where the detector array is configured to detect a ray emitted from the distributed ray source and passing through the scanned object, and to generate projection data according to the detected ray, wherein the detector array is configured to generate first projection data in response to the first ray beam passing through the scanned object, and generate second projection data in response to the second ray beam passing through the scanned object; and an image reconstruction device, where the image reconstruction device is configured to generate a computed tomography image of the scanned object according to the first projection data and the second projection data. The distributed ray source is configured to rotate around a first axis when the scanned object is scanned, and the first axis is parallel to the conveying direction. In the CT scanning system, a distributed ray source technology and a rotary spiral CT scanning technology are combined into a whole, the distributed ray source is used for replacing a single-point ray source in the conventional spiral CT scanning system, and the CT scanning with high scanning speed may be implemented under the condition that the detector row number is not increased.

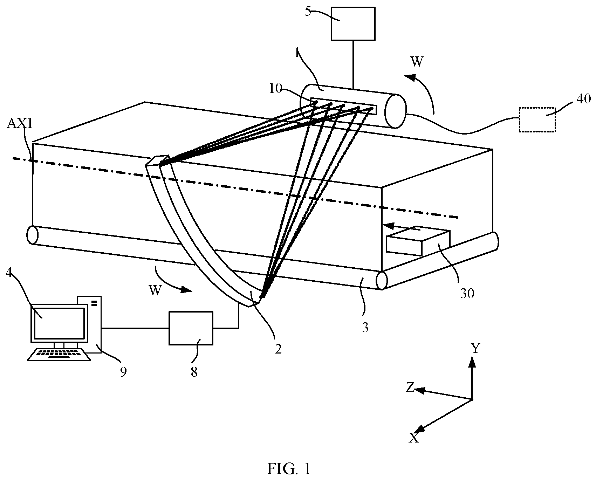

FIG. 1 is a schematic structural view of a CT scanning system according to some exemplary embodiments of the present disclosure. As shown in FIG. 1, a CT scanning system may include: a conveying device 3 configured to move the scanned object 30 in the scanning channel 31 in a predetermined conveying direction Z; a distributed ray source 1, where the distributed ray source 1 includes m target points 10, the m target points 10 are configured to be activated in a predetermined order to emit ray beams, m is a positive integer greater than or equal to 2, the ray beams emitted by at least one of the m target points is modulated into a first ray beam having a first energy, and the ray beams emitted by at least another of the m target points is modulated into a second ray beam having a second energy, where the first energy is lower than the second energy; a detector array 2, where the detector array 2 is configured to detect a ray emitted from the distributed ray source 1 and passing through the scanned object 30, and to generate projection data according to the detected ray, wherein the detector array 2 is configured to generate first projection data in response to the first ray beam passing through the scanned object 30, and generate second projection data in response to the second ray beam passing through the scanned object 30; and an image reconstruction device 4, where the image reconstruction device 4 is configured to generate a computed tomography image of the scanned object 30 according to the first projection data and the second projection data.

For example, the conveying device 3 may be implemented in a belt conveying manner, a chain conveying manner, a gear conveying manner, or other conveying manners, and the embodiment of the disclosure is not limited thereto. For example, the scanned object 30 is placed on the conveying surface of the conveying device 3.

In an embodiment of the present disclosure, a CT scanning system uses a distributed ray source 1. The distributed ray source 1 includes a plurality of target points 10 for emitting ray beams. The plurality of target points 10 may be arranged according to a predetermined geometric shape and a predetermined interval distance, for example, the predetermined geometric shape may include a straight line, an arc line, a plane, a curved surface, and the like.

Specifically, in an embodiment of the present disclosure, the distributed ray source 1 includes an X-ray generating tube having a plurality of target points, or optionally, the distributed ray source 1 includes a plurality of X-ray generating tubes having a single target point.

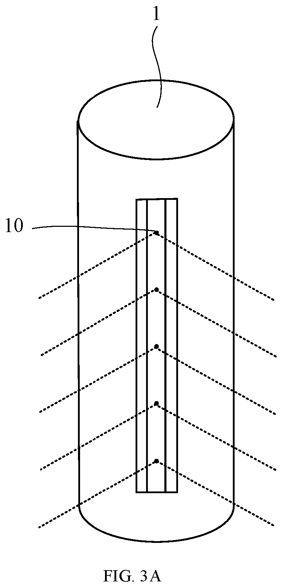

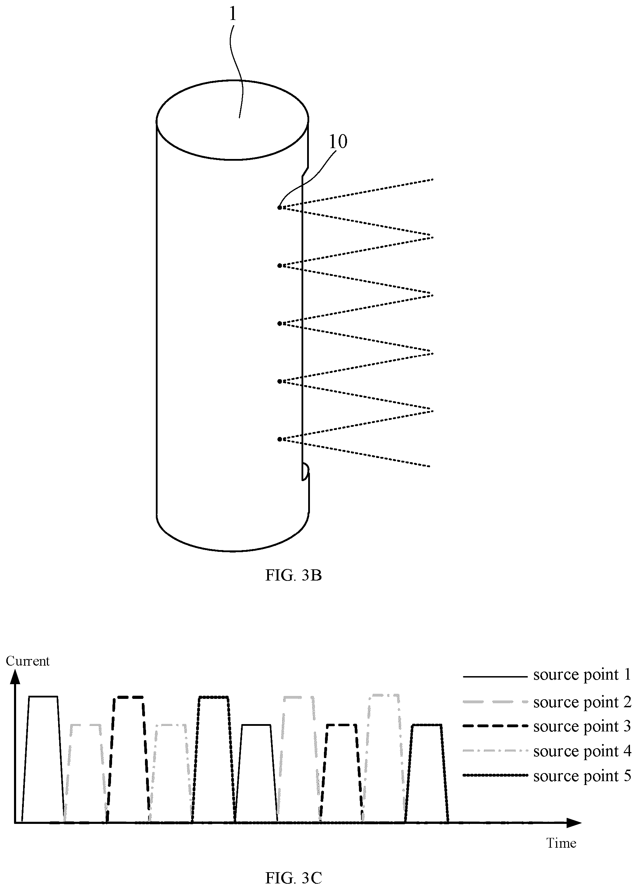

FIG. 3A is a schematic structural diagram of a distributed ray source according to some exemplary embodiments of the present disclosure, and FIG. 3B is a schematic structural diagram of the distributed ray source in FIG. 3A viewed from another perspective. With reference to FIG. 3A and 3B, the distributed ray source 1 includes an X-ray generating tube having m target points 10. The m target points 10 are configured to be activated to emit a ray beam in a predetermined sequence, the ray beams emitted by the plurality of target points 10 are schematically illustrated in FIGS. 3A and 3B.

The X-ray generating tube may be, for example, an X-ray generator using a cold cathode carbon nanotube. Specifically, the X-ray generating tube may include a cold cathode carbon nanotube emission unit, an accelerator system, a target material, and a cooling system. The cold cathode carbon nanotube emission unit may include a plurality of cold cathode carbon nanotubes as electron emission sources. The carbon nanotubes are fixed in the emission cell by a suitable preparation process and are connected to the electron source circuit. The emission characteristics of the cold cathode carbon nanotubes enable them to provide stable electron emission and generate high intensity electron beams. The accelerator system is used for accelerating electron beams emitted by the cold cathode carbon nanotubes. It may include a set of electron lenses and an electric field accelerator that controls the focusing and acceleration of the electron beam by adjusting the parameters of the electric field and the lenses. The target material is the target of the electron beam impact that strikes the target material to generate X-ray radiation. For example, the target material may be a high atomic number metal, such as tungsten or molybdenum. When the electron beam emitted by the cold cathode carbon nano tube impacts the target material, characteristic X rays and continuous spectrum X rays are generated. Since a large amount of heat is generated during the X-ray generation, a cooling system is required to ensure stable operation of the system. The cooling system may adopt an air cooling or liquid cooling mode, and heat is effectively dissipated through the heat dissipation device.

It should be noted that, the distributed ray source is described here by taking an X-ray generating tube using a cold cathode carbon nanotube as an example, however, the embodiments of the present disclosure are not limited to this form of X-ray generator, and distributed ray sources with other suitable structures may be applied to the CT scanning system provided in the embodiments of the present disclosure.

Referring to FIG. 1, in an embodiment of the present disclosure, the CT scanning system further includes a voltage supply device 40, where the voltage supply device 40 is configured to provide voltages to the m target points of the distributed ray source respectively; the voltage supply device 40 is configured to: supply a first voltage to at least one of the m target points and supply a second voltage to at least another one of the m target points, where the first voltage is lower than the second voltage.

For example, the first and second voltages supplied by the voltage supply device 40 are different, so that the ray beams emitted from at least one of the m target points is modulated into a first ray beam having a first energy, and the ray beams emitted from at least another one of the m target points is modulated into a second ray beam having a second energy, such that the first energy is lower than the second energy.

FIG. 3C schematically shows a waveform diagram of a current used to control a beam output of a distributed ray source. As shown in FIG. 3C, the schematically illustrated current may be applied to a distributed ray source having 5 target points. The 5 target points of the distributed ray source emit beams alternately according to the timing shown in FIG. 3C, and each time the beam is emitted, the detector and the data acquisition system complete a data acquisition.

It should be noted that, in the present disclosure, a distributed ray source having 5 target points is schematically illustrated, and the number of target points of the distributed ray source is not particularly limited by the embodiments of the present disclosure, and in other embodiments, the distributed ray source 1 may include a smaller number (e.g., 4, 3) or a larger number (e.g., 6, 9, 10) of target points.

Referring back to FIG. 1, the CT scanning system may further include a controller 5, the controller 5 is configured to control at least one of the following aspects of the distributed ray source 1: the activation moment of the m target points 10, the duration of the ray beams emitted by the m target points 10, the intensity of the ray beams emitted by the m target points 10, and the energy of the ray beams emitted by the m target points 10.

In the embodiment of the present disclosure, the ray beam-emitting of each target point may be controlled according to a predetermined timing sequence, and particularly, the duration, intensity and energy of the ray beams emitted by each target point may be independently controlled.

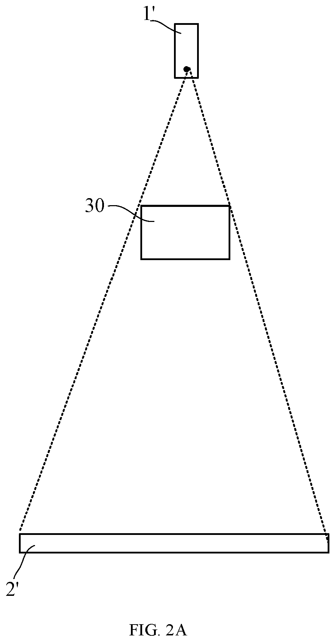

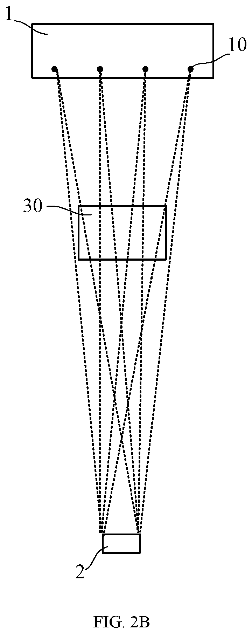

FIG. 2A is a scanning schematic diagram of a conventional CT scan, and FIG. 2B is a scanning schematic diagram of a CT scanning using a distributed ray source according to an embodiment of the present disclosure.

As shown in FIG. 2A, in a conventional CT scan, a ray beams emitted from a single ray source 1' passes through an object to be detected, and is detected by a detector 2' to form projection data. The single ray source corresponds to a multi-row detector or an area array detector.

As shown in FIG. 2B, in an embodiment of the present disclosure, the detector array may include n1 rows of detectors or an area array detector, n1 is a positive integer greater than or equal to 1; the distributed ray source 1 includes m target points, m is a positive integer greater than or equal to 2, and m is greater than n1. That is, in embodiments of the present disclosure, the number of target points of the distributed ray source is greater than the number of rows of the detector array.

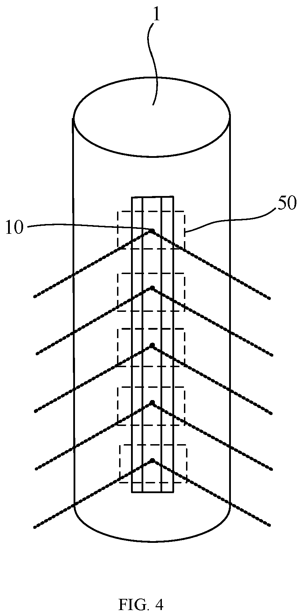

FIG. 4 is a structural schematic diagram of yet another distributed ray source according to some exemplary embodiments of the present disclosure.

As shown in FIG. 4, in the embodiment of the present disclosure, the voltage supply device 40 may provide voltages to the m target points of the distributed ray source respectively, which may be substantially equal, for example, the first voltage may be substantially equal to the second voltage. In this case, the CT scanning system further includes at least one ray filter 50, and the at least one ray filter 50 is arranged on the beam path of at least one target point of the m target points, and is configured to modulate the ray beams emitted by the at least one target point of the m target points into a first ray beam having a first energy.

Specifically, by changing the material type of the ray filter 50, a group of ray filters 50 made of at least two materials may be placed at the m target point ray beam outlets, such as copper and tin or aluminum and tantalum. After the ray passes through different material filters, h target points are modulated into a first ray beam having a first energy, and j target points are modulated into a second ray beam having a second energy, so that the first energy is lower than the second energy, where h and j are positive integers greater than or equal to 1 and less than m, respectively, and h + j = m.

In the CT scanning using the distributed ray source according to the embodiment of the present disclosure, the distributed ray source having a plurality of target points sequentially emits a first ray beam having a first energy and a second ray beam having a second energy, and the first ray beam and the second ray beam pass through the object to be detected, and then are detected by the detector to form first projection data and second projection data, and the image reconstruction device 4 generates a computed tomography image of the object to be scanned 30 according to the first projection data and the second projection data. A distributed ray source having a plurality of target points corresponds to a smaller number of detectors. The "smaller number" herein may include the following cases: the row number of the detector is reduced compared with that of the conventional CT scanning; or the area of the area array detector is reduced compared to conventional CT scanning. In addition, the CT scanning using the distributed ray source according to the embodiment of the present disclosure also has the advantages of suppressing cone angle artifacts, reducing scattering influence, optimizing dose, and the like.

The principle of dual-energy CT image reconstruction is described in detail below.

Firstly, the object to be detected is scanned by dual-energy ray to obtain dual-energy projection data.

Next, the projection value of the basis material coefficient corresponding to the dual-energy projection data is calculated according to a preestablished lookup table or by a method of solving an equation system. The method for creating the lookup table includes the steps of selecting two base materials, calculating projection values of dual-energy rays passing through the two materials under different thicknesses, and obtaining the lookup table according to the relation between the high-energy projection values and the low-energy projection values and different thickness combinations. The method for solving the equation set by analysis is to obtain corresponding thickness combinations by solving the high-low energy projection equation set under the base material decomposition by utilizing the actually obtained high-low energy projection values.

Then, from the projection values of the basis material, a distribution image of the basis material coefficients may be obtained. The atomic number and the characteristic density image of the object to be detected and the attenuation coefficient image of the object to be detected under any energy may be obtained from the distribution of the base material coefficients, so that the object to be detected is subjected to material classification and automatic identification.

That is, in the embodiments of the present disclosure, the distributed X-ray source 1 may be an energy-adjustable single energy accelerator. The ray beams emitted by at least one of the m target points is modulated into a first ray beam having a first energy, and the ray beams emitted by at least another of the m target points is modulated into a second ray beam having a second energy. The projection image data obtained in this way is subjected to dual-energy CT reconstruction, and the obtained image information of the scanned object 30 includes information such as a high-energy attenuation coefficient, a low-energy attenuation coefficient, an atomic number, and an electron density, so that the object to be detected is subjected to material classification and automatic identification.

The multi-source spiral CT system of the present disclosure includes a distributed X-ray source, a detector, a slip ring, a rack, an object conveying device, a data acquisition system and a data processing system. The distributed X-ray source is the main difference of the present disclosure from the conventional spiral CT.

Referring back to FIG. 1, the CT scanning system may further include a data acquisition system 8 and a data processing system 9. For example, the data acquisition system 8 may be configured to acquire signal triggers and has the data transmission functions. Before one target point of the ray source emits a beam, the data acquisition system 8 sends out an acquisition signal, and the detector starts integration (or counting); after the target point stops, the data acquisition system 8 sends out a stop acquisition signal and the detector completes integration (or counting). The data acquisition system 8 transmits the result of this acquisition of the detector to a subsequent data processing system 9. That is, the data acquisition of the detector and the target point beam-out are synchronous.

The data processing system 9 is configured for correction, reconstruction, automatic recognition, image processing and display of the acquired data. The correction includes background correction, gain correction and the process of taking negative logarithm transformation into line integral. The reconstruction may adopt an analytic algorithm or an iterative algorithm to calculate the attenuation coefficient or the CT number of the scanned object. If dual-energy data or energy spectrum data are acquired, a dual-energy reconstruction algorithm or an energy spectrum reconstruction algorithm may be used for calculating the density and the atomic number information of the scanned object and the selected basis material coefficient information. And the automatic identification is to perform operations such as segmentation, statistics, classification and the like on the reconstruction result, compare the reconstruction result with the characteristic database, determine whether the scanned object has components which accord with the inspection characteristics, and give a determination conclusion. And the image processing and displaying means that the reconstruction result and the automatic identification result are displayed on a screen in the form of a tomography image or a three-dimensional rendering image for a user to observe and judge.

Referring to FIG. 1, the distributed ray source 1 is configured to rotate around a first axis AX1 when the scanned object 30 is scanned, and the first axis AX1 is parallel to the conveying direction Z.

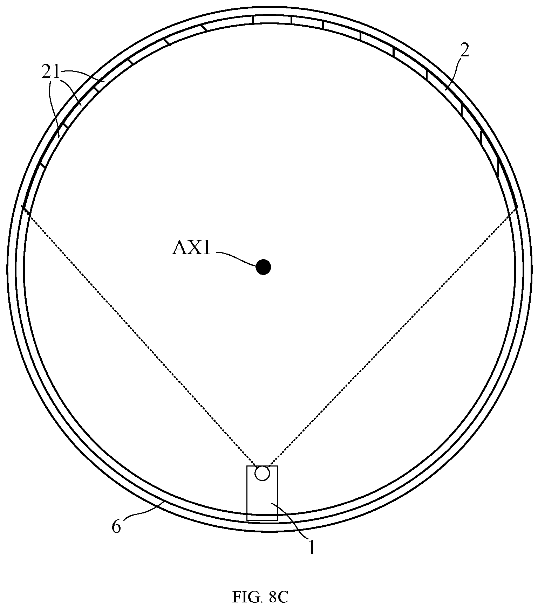

In some exemplary embodiments, the CT scanning system further includes a support member 6 (refer to FIG. 8C) for supporting the distributed ray source 1 and the detector array 2. The support member 6 is configured to drive the distributed ray source 1 and the detector array 2 to rotate around the first axis AX1 when the scanned object 30 is scanned.

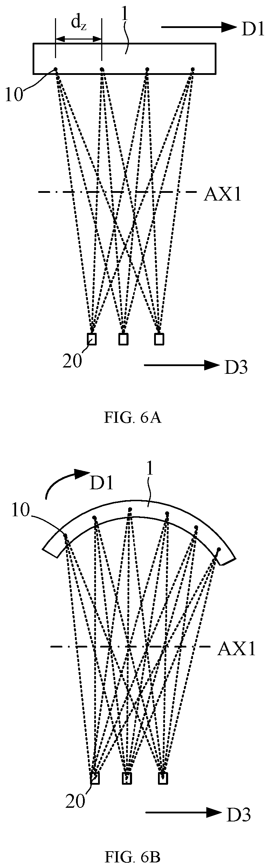

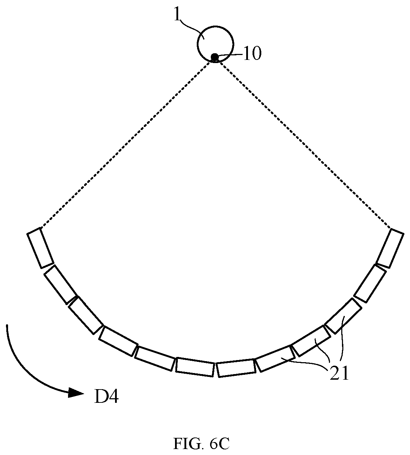

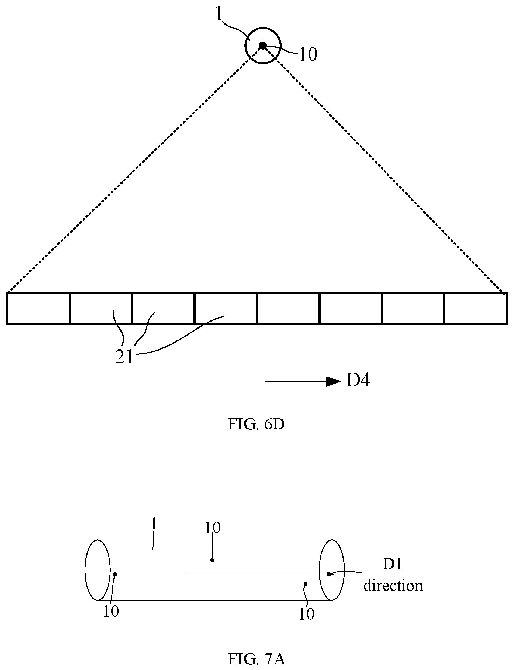

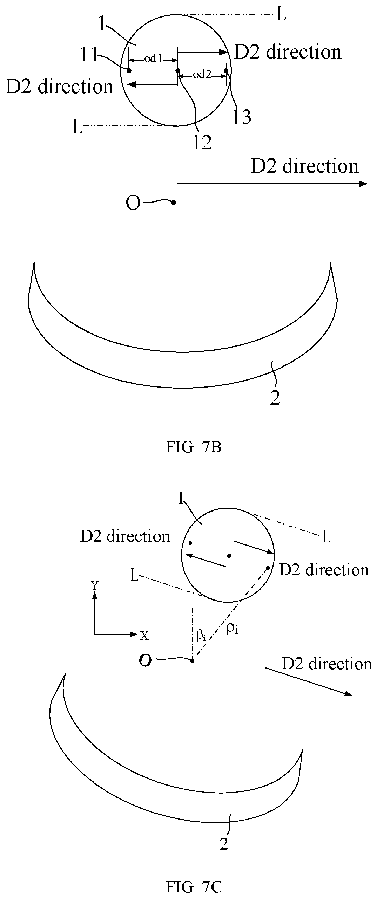

Herein, for convenience of description, for the CT scanning system, an XYZ spatial coordinate system is established, as shown in FIG. 1, an X direction is a width direction of a scanning channel, a Y direction is a height direction of the scanning channel, and a Z direction is a conveying direction of a scanned object in the scanning channel. In this XYZ space, there is a rotational movement of the ray source 1 or the detector array 2, the direction of rotation about the first axis AX1 being indicated by the W direction. For a distributed ray source, establishing D1-D2 directions, in the embodiment shown in FIG. 1, a first arrangement direction D1 (shown in FIG. 7A) is parallel to the direction Z for representing the alignment of the plurality of target points of the distributed ray source in the direction Z; as shown in FIG. 7B and 7C, the tangential direction D2 is a direction extending along a tangent to the rotation direction W of the distributed ray source 1, i.e. the tangential direction D2 represents a direction perpendicular to the radial direction of the ray source in each rotation state. For the detector array, a direction D3-D4 is established, wherein, as shown in FIGS. 6A and 6B, the second arrangement direction D3 is parallel to the direction Z for indicating the alignment of the plurality of rows of detectors in the direction Z; as shown in FIG. 6C and 6D, the third arrangement direction D4 is perpendicular to the direction Z for indicating an arrangement of a plurality of detector modules of a row of detectors in a direction perpendicular to the direction Z.

It should be noted that the various directions are only used for convenience of describing the embodiments of the present disclosure, and are not intended to limit the scope of the present disclosure.

For example, the support member 6 may include a slip ring structure for a CT device. Specifically, the slip ring structure may include a fixed portion, a rotating portion, a contactor, and a conductive ring. The fixed portion, also referred to as a stationary ring, may be mounted on the fixed portion of the CT device (e.g., a gantry or a base of a CT scanning system). The rotating portion is also called rotating ring. The distributed ray source 1 and the detector array 2 are connected to the rotating portion. The fixed ring is the fixed end of the slip ring, and the rotating ring is the rotating end of the slip ring. The contactor is a set of electrically conductive brushes or contact blades fixed to the rotating portion. They contact the metal ring of the rotating portion, thereby establishing an electrical connection. The contactor may be made of a conductive material (e.g., carbon) having good conductivity and wear resistance. The conductive ring is a set of metal rings fixed to the fixed portion. They are in contact with the contactor of the rotating portion, forming an electrical connection. The conductive ring may be made of a highly conductive metal (e.g., copper) to ensure good electrical transmission.

In embodiments of the present disclosure, the detector array 2 may include various types of detectors suitable for use in a CT scanning system. For example, the detector array 2 may include a single row detector, a multi-row detector, or an area array detector, divided by the number of rows and the geometric detector efficiency. Divided in the way the X-ray signals are acquired, the detector array 2 may include energy deposition type detectors or photon counting detectors. The detector array 2 may include a single-energy detector, a dual-energy detector, or a spectral detector, distinguished from detecting energy.

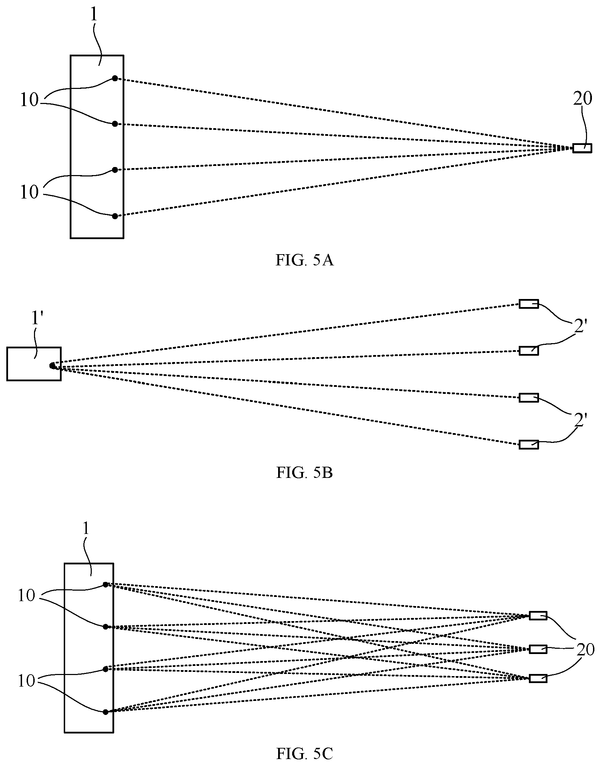

FIG. 5A is a schematic structural diagram of a combination of a source and a detector of a CT scanning system according to an embodiment of the present disclosure, in which a distributed ray source and a single row of detectors are schematically illustrated. FIG. 5B is a schematic diagram of a combination of a source and a detector of a conventional CT scanning system, in which a single-target ray source and a plurality of rows of detectors are schematically illustrated.

With reference to FIG. 1 and 5A, detector array 2 includes a single row of detectors 20. For example, in the embodiment shown in FIG. 5A, the distributed ray source 1 includes 4 target points 10. In the embodiment of FIG. 5B, in a conventional CT scanning system, a single target point source 1' and 4 rows of detectors 2' are provided. With reference to FIG. 5A and 5B, in a plane perpendicular to the first axis AX1, the resolution of the ray scanning provided by the combination of a distributed ray source 1 having four target points 10 and a single row of detectors 20 in the region where the scanned object 30 is located is basically the same as the resolution of the ray scanning provided by the combination of ray source 1' having a single target point and four rows of detectors 2' in the region where the scanned object 30 is located. Accordingly, the scanning combination of the distributed ray source 1 having 4 target points 10 and the single row of detectors 20 may obtain the same image effect with the and the scanning combination of the ray source 1' having a single target point and the 4 rows of detectors 2'. In this case, the detector cost may be reduced to 1/4 relative to a 4 rows of detectors 2'.

That is, the CT scanning system according to the embodiment of the present disclosure may include the distributed ray source 1 having m target points and the single-row detector 20, and in a plane perpendicular to the first axis AX1, the resolution of the ray scanning provided by the combination of the distributed ray source 1 having m target points and the single-row detector 20 in the region where the scanned object 30 is located is substantially the same as the resolution of the ray scanning provided by the combination of the ray source 1' with the single target point and the m-row detector 2' in the region where the scanned object 30 is located, and the detector cost may be reduced to 1/m.

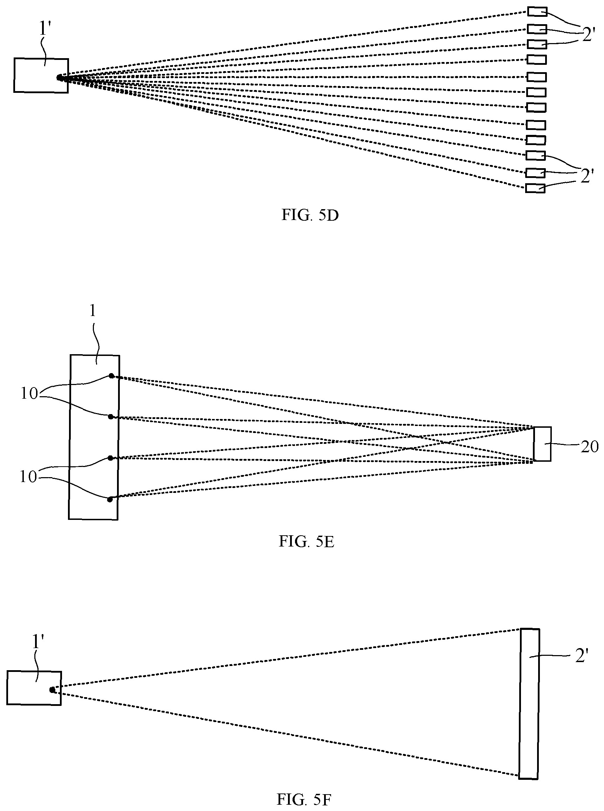

FIG. 5C is a schematic structural diagram of a combination of a source and a detector of a CT scanning system according to an embodiment of the present disclosure, in which a distributed ray source and a plurality of rows of detectors are schematically illustrated. FIG. 5D is a schematic diagram of a combination of a source and a detector of a conventional CT scanning system, in which a ray source having a single target point and further rows of detectors are schematically illustrated.

Referring to FIGS. 1 and 5C, the detector array 2 includes n rows of detectors, where n is a positive integer greater than or equal to 2. For example, in the embodiment shown in FIG. 5C, the distributed ray source 1 includes 4 target points 10, i.e. m = 4; the detector array 2 includes 3 rows of detectors 20, i.e. n = 3. In the embodiment of FIG. 5D, in a conventional CT scanning system, a ray source 1' having single target point and 12 rows of detectors 2' are provided. With reference to FIG. 5C and 5D, in a plane perpendicular to the first axis AX1, the resolution of the ray scanning provided by the combination of the distributed ray source 1 having 4 target points 10 and the 3 rows of detectors 20 in the region where the scanned object 30 is located is substantially the same as the resolution of the ray scanning provided by the combination of the source 1' having a single target point and the 12 rows of detectors 2' in the region where the scanned object 30 is located. Accordingly, the scanning combination of the distributed ray source 1 having 4 target points 10 and 3 rows of detectors 20 may obtain substantially the same image effect as the scanning combination of the ray source 1' having a single target point and 12 rows of detectors 2'. In this case, the detector cost may be reduced to 1/4 relative to a 12-row detector 2'.

That is, the CT scanning system according to the embodiment of the present disclosure may include the distributed ray source 1 having m target points and the n rows of detectors 20, and in a plane perpendicular to the first axis AX1, the combination of the distributed ray source 1 having m target points and the n rows of detectors 20 provides the resolution of ray scanning in the region where the scanned object 30 is located, which is substantially the same as the resolution of the ray scanning provided by the combination of the ray source 1' having a single target point and the p rows of detectors 2' (p = m×n) in the region where the scanned object 30 is located, while the detector cost may be reduced to 1/m.

In the embodiment shown in FIG. 5C, due to the combined use of the distributed ray source and the plurality of rows of detectors, the cone angle of the ray beam is reduced, and the influence of the cone angle effect on the reconstructed image is also reduced, so that the quality of the reconstructed image may be improved. Furthermore, the distributed ray source may be combined with a plurality of rows of detectors which are far away from each other, so that the coverage of ray beams in the moving direction (namely, the Z direction) of the object may be increased, and the scanning speed is improved.

FIG. 5E is a schematic structural diagram of a combination of a source and a detector of a CT scanning system in which a distributed ray source and an area array detector are schematically illustrated according to an embodiment of the present disclosure; FIG. 5F is a schematic diagram of a combination of a source and a detector of a conventional CT scanning system in which a single-target ray source and an area array detector are schematically illustrated.

With reference to FIG. 1 and 5E, the detector array 2 includes an area array detector 20, which may include a plurality of rows of detector modules. For example, in the embodiment shown in FIG. 5E, the distributed ray source 1 includes 4 target points 10, i.e. m = 4; the area array detector includes 6 rows of detector modules. In the embodiment of FIG. 5F, in a conventional CT scanning system, a ray source 1' having a single target point and an area array detector 2' are provided, and the area array detector 2' includes 24 rows of detector modules. With reference to FIG. 5E and 5F, in a plane perpendicular to the first axis AX1, the combination of the distributed ray source 1 having 4 target points 10 and the area array detector 20 provides the resolution of ray scanning in the region where the scanned object 30 is located, which is substantially the same as the resolution of ray scanning provided by the combination of the ray source 1' having a single target point and the area array detector 2' in the region where the scanned object 30 is located. Accordingly, the scanning combination of the distributed ray source 1 having 4 target points 10 and the area array detector 20 may obtain the substantially the same image effect as the scanning combination of the ray source 1' having a single target point and the area array detector 2'. In this case, the detector cost may be reduced to 1/4 with respect to the area array detector 2'.

That is, the CT scanning system according to the embodiment of the present disclosure may include the distributed ray source 1 having m target points and the area array detector 20 having n rows of detector modules, and within a plane perpendicular to the first axis AX1, the combination of the distributed ray source 1 having m target points and the area array detector 20 provides a resolution of a ray scanning in the region where the scanned object 30 is located, which is substantially the same as a resolution of a ray scanning provided by the combination of the ray source 1' having a single target point and the area array detector 2' having p rows of detector modules (p = m× n) in the region where the scanned object 30 is located, and the detector cost may be reduced to 1/m.

In the embodiment shown in FIG. 5E, the distributed ray source is combined with the area array detector having a smaller area, so that the cost of the area array detector may be reduced and high-resolution imaging may be implemented.

FIG. 6A to 6D schematically show some arrangements of a source and a detector according to some exemplary embodiments of the present disclosure.

FIG. 6A is an exemplary arrangement of the source and the detector as viewed in the Y direction. As shown in FIG. 6A, in some embodiments, the distributed ray source 1 may include m target points 10, the m target points 10 are spaced apart in a first arrangement direction D1, and the first arrangement direction D1 may be substantially parallel to the first axis AX1. For example, the m target points 10 may be spaced along a straight line parallel to the first arrangement direction D1. Optionally, the m target points 10 may be arranged at equally spaced intervals. For example, in the distributed ray source, m target points are arranged at equal intervals along a first arrangement direction by a preset interval distance dz.

With continued reference to FIG. 6A, the n rows of detectors 20 may be spaced apart in a third arrangement direction D3, and the third arrangement direction D3 may be substantially parallel to the first axis AX1. For example, the n rows of detectors 20 may be arranged at equally spaced intervals.

FIG. 6B is another exemplary arrangement of the source and the detector as viewed in the Y-direction. As shown in FIG. 6B, in some embodiments, the distributed ray source 1 may include m target points 10, the m target points 10 are spaced apart in a first arrangement direction D1, and the first arrangement direction D1 may be an arc direction. For example, the m target points 10 may be arranged at intervals along an arc or a curve. Optionally, the m target points 10 may be arranged at equal intervals along an arc or curve. For example, in the distributed ray source, m target points are arranged at equal intervals along a first arrangement direction by a preset interval distance dz.

With continued reference to FIG. 6B, the n rows of detectors 20 may be spaced apart in a third arrangement direction D3, and the third arrangement direction D3 may be substantially parallel to the first axis AX1. For example, the n rows of detectors 20 may be arranged at equally spaced intervals.

FIG. 6C is an exemplary arrangement of a source and a detector as viewed in the Z-direction. As shown in FIG. 6C, in some embodiments, the detector array 2 may include a single row of detectors, a plurality of rows of detectors, or an area array detector. For a single row of detectors, a plurality of rows of detectors or an area array of detectors, each row of detectors or area array of detectors includes a plurality of detector modules 21, the plurality of detector modules 21 of at least one row of detectors are arranged continuously in a fourth arrangement direction D4 in a plane perpendicular to the first axis. The fourth arrangement direction D4 may be an arc direction. That is, a plurality of detector modules 21 may be arranged in series along an arc or curve.

In this embodiment, the m target points 10 may be spaced in the first arrangement direction D1, and the first arrangement direction D1 may be a linear direction or a curved direction.

FIG. 6D shows another exemplary arrangement of the source and the detector as viewed in the Z-direction. As shown in FIG. 6D, in some embodiments, the detector array 2 may include a single row of detectors, a plurality of rows of detectors, or an area array detector. For a single row of detectors, a plurality of rows of detectors or an area array of detectors, each row of detectors or area array of detectors includes a plurality of detector modules 21, the plurality of detector modules 21 of at least one row of detectors are arranged continuously in a fourth arrangement direction D4 in a plane perpendicular to the first axis. The fourth arrangement direction D4 may be a straight direction perpendicular to the first axis AX1. That is, a plurality of detector modules 21 may be arranged in series along a straight line.

In this embodiment, the m target points 10 may be spaced in the first arrangement direction D1, and the first arrangement direction D1 may be a linear direction or a curved direction.

It should be noted that FIG. 6A to 6D exemplarily show some arrangements of a combination of a source and a detector, and the embodiments of the present disclosure are not limited to the several arrangements listed herein, and in a case of no conflict, the several arrangements listed in FIG. 6A to 5D may be combined with each other, and the embodiments of the present disclosure may further include other suitable arrangements.

FIG. 7A to 7C respectively schematically illustrate an arrangement of a plurality of target points of a distributed ray source in a CT scanning system according to some exemplary embodiments of the present disclosure, wherein FIG. 7A is a perspective view of the distributed ray source, FIG. 7B is a schematic view of the source and the detector in a rotation state, and FIG. 7C is a schematic view of a source and a detector in another rotation state. FIG. 7B and 7C are schematic views of the source and the detector respectively as viewed in the Z direction.

With reference to FIG. 1, 7A to 7C, in some exemplary embodiments of the present disclosure, in the distributed ray source 1, at least two target points of the m target points 10 are arranged offset in a tangential direction D2, wherein the tangential direction D2 is a direction extending along a tangent to the rotation direction W of the distributed ray source 1. For example, in the rotation state shown in FIG. 7B, the tangent line L extends exactly in the horizontal direction; in the rotated state shown in FIG. 7C, the tangent line L extends in an oblique direction.

As shown in FIG. 7A to 7C, 3 target points among the m target points 10 are schematically shown, and for convenience of description, the 3 target points are respectively labeled as a first target point 11, a second target point 12, and a third target point 13.

At least two target points (e.g., the first target point 11 and the second target point 12) of the m target points are offset in the tangential direction D2 by a first offset od 1. At least two target points (e.g., the second target point 12 and the third target point 13) of the m target points are offset in the tangential direction D2 by a second offset od 2.

In some exemplary embodiments, the first offset od1 and the second offset od2 are substantially equal.

It should be noted that, in the present disclosure, unless otherwise specified, "substantially equal" includes the case where two amounts are strictly equal or where two amounts are equal in an engineering sense, for example, when a ratio between two amounts is in a range of 0.8 to 1.2, the two amounts may be considered to be substantially equal.

As shown in FIG. 7A to 7C, the coordinates of the i-th target point mi in the XYZ coordinate system are (xᵢ, yᵢ, zᵢ). For example, when the m target points are arranged at equal intervals in the first arrangement direction D1, Z coordinates zᵢ of the m target points are an arithmetic progression with a tolerance dz, where dz is an equal interval. ρᵢ is the distance from the target point mi to the rotation center O (i.e., the orthographic projection point of the first axis AX1 on the plane perpendicular to the Z direction). βᵢ is the rotation angle of the target point mi, i.e., the angle of the line connecting the target point mi and the rotation center O with respect to the direction Y. The coordinates xᵢ, yᵢ of the i-th target mi in the XYZ coordinate system may be calculated by:
${x}_{i} = {ρ}_{i} sin {β}_{i} ,$
${y}_{i} = {ρ}_{i} cos {β}_{i} ,$

In the embodiments of the present disclosure, a part of target points 10 are arranged in a staggered manner in the tangential direction, that is, ρᵢ and βᵢ are designed to have different values, so that the part of target points 10 are located at different positions on a plane perpendicular to the Z direction. Through the dislocation arrangement, the ray projection data has lower information redundancy and higher information quality.

In the embodiment of the present disclosure, all m target points 10 may be arranged in a staggered manner in the tangential direction, that is, any two target points of the m target points 10 are arranged in an offset manner in the tangential direction D2. Through the staggered arrangement, the ray projection data further has lower information redundancy and higher information quality.

Further, in the embodiment of the present disclosure, the offset between adjacent target points 10 is determined according to the rotation speed ω of the distributed ray source, the moving speed v of the conveying device, and the interval distance dz of the target points in the Z direction. That is, the first offset od1 and the second offset od2 are determined according to the rotation speed ω of the distributed ray source, the moving speed v of the conveying device, and the interval distance dz of the target point in the first arrangement direction D1 (parallel to the Z direction).

The inventors found that the difference Δβ between the rotation angles of adjacent target points 10 should be between integer multiples vω/dz. That is, Δβ=kvω/dz, where k is a non-integer. Therefore, in the process of continuous spiral scanning, ray beams emitted by a plurality of target points which are arranged in a staggered mode in the tangential direction may irradiate scanned objects from different angles, and then projection data detected by the detector may contain less redundancy information, so that the image quality may be improved.

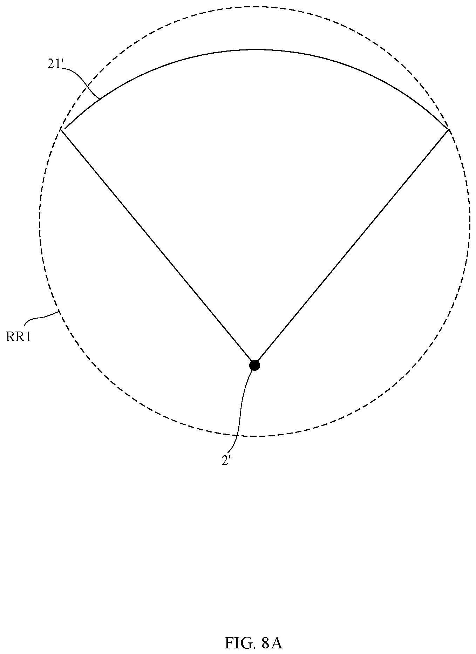

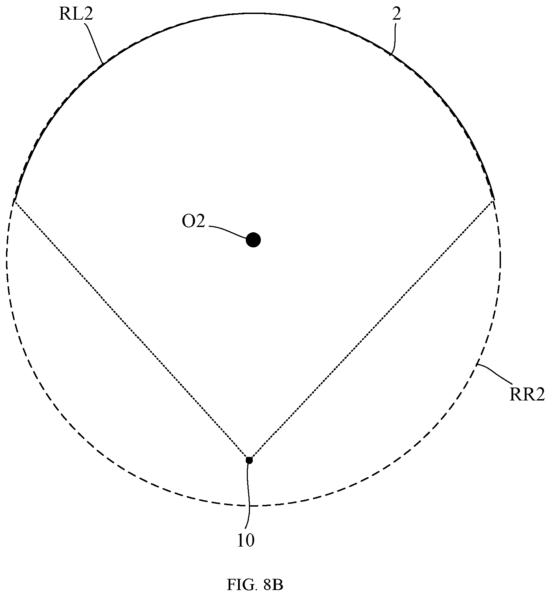

FIG. 8A schematically illustrates an arrangement of a source and a detector in a conventional CT scanning system, FIG. 8B schematically illustrates an exemplary arrangement of a source and a detector in the CT scanning system according to an embodiment of the present disclosure, and FIG. 8C schematically illustrates an installation structure of a source and a detector in the CT scanning system according to an embodiment of the present disclosure.

As shown in FIGS. 8B and 8C, each row of detectors 20 may include a plurality of detector modules 21, and the plurality of detector modules 21 of at least one row of detectors are arranged continuously along an arc line RL2 in a plane perpendicular to the first axis AX1. The arc line RL2 has a center O2. In a plane perpendicular to the first axis AX1, the at least one target point 10 is located offset from a center O2 of the arc line. That is, in the embodiment of the present disclosure, the detector modules 21 are not arranged along an arc centered on the target point. That is, the detector may be arranged non-centripetally with respect to the target point of the source.

As shown in FIG. 8A, in the conventional CT scanning system, the detector needs to be arranged centripetally with respect to the target point of the ray source, i.e. the detector module 21' is arranged along an arc line with the target point 2' as the center.

The rotation ranges RR1 and RR2 of the slip ring are schematically shown in FIG. 8A and 8B, respectively. Referring to FIG. 8A and 8B, in the embodiment of the present disclosure, compared to the rotation range RR1 of the slip ring in FIG. 8A, the rotation range RR2 of the slip ring in the embodiment of the present disclosure is narrowed, that is, the rotation radius of the slip ring is reduced, so that the size of the CT scanning device may be reduced, and the device weight may be reduced.

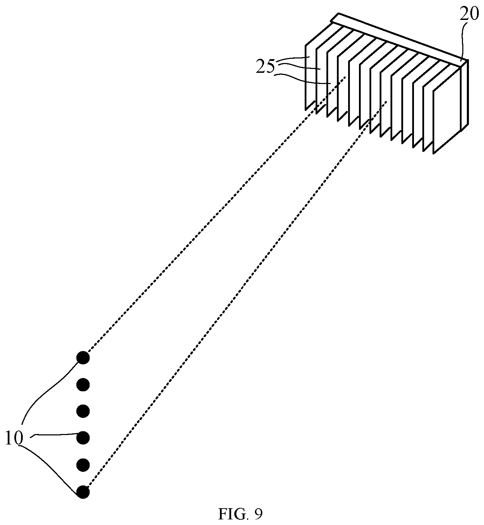

In the embodiment of the present disclosure, since the position of each target point of the distributed ray source relative to the detector in the fan-beam plane is the same, a post collimator 25 along the fan-angle direction may be installed in front of the detector 20 to suppress the interference of the scattered signals, as shown in FIG. 9. In some exemplary embodiments of the present disclosure, the CT scanning system may further include a post collimator 25, the post collimator 25 being located at a side of the detector array 2 facing the distributed ray source 1.

Similar to FIG. 5C and 5D, the post collimator 25 includes a plurality of sub-collimators, which are continuously arranged along a straight line or an arc line in a plane perpendicular to the first axis.

In embodiments of the present disclosure, an anti-scatter post collimator is used to suppress the effect of scattered photons on image quality. The inventor finds that for a multi-row detector and an area array detector, the post collimator is complex in structure, high in processing difficulty and high in cost. In the embodiment of the present disclosure, since the distributed ray source is applied to the spiral CT scanning system, a single-row detector may be used, and accordingly, the structural complexity of the post collimator may be reduced, the processing difficulty thereof may be reduced, and the cost thereof may be reduced.

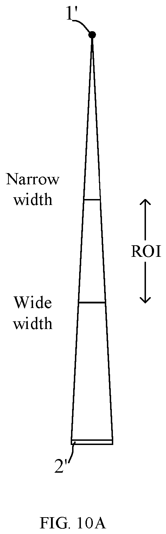

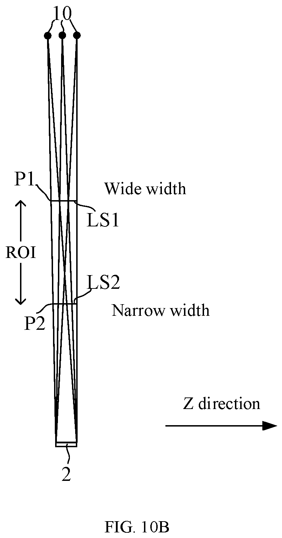

FIG. 10A schematically illustrates a scanning range of a conventional CT scanning system, and FIG. 10B schematically illustrates a scanning range of a CT scanning system according to some exemplary embodiments of the present disclosure.

As shown in FIG. 10A, the conventional CT scanning system includes a ray source 1' having a single target point in a region of interest ROI where the scanned object 30 is located. The ray coverage closer to the ray source 1' is narrower, and the ray coverage closer to the detector array 2' is wider, as viewed in the Z direction.

As shown in FIG. 10B, the ray beams formed by the m target points 10 of the distributed ray source 1 form a scanning range in the region of interest ROI where the scanned object 30 is located, the scanning range includes a first position P1 and a second position P2, the first position P1 is closer to the distributed ray source 1 than the second position P2, and the second position P2 is located between the first position P1 and the detector array 2. The scanning range is formed with a first straight line segment LS1 parallel to the conveying direction at a first position P1, the scanning range is formed with a second straight line segment LS2 parallel to the conveying direction at a second position P2, and the width of the first straight line segment LS1 is larger than that of the second straight line segment LS2.

In some exemplary embodiments of the present disclosure, the ratio of the width of the first straight-line segment LS1 to the width of the second straight-line segment LS2 is greater than 1 and equal to or less than 1.5.

That is, in the embodiments of the present disclosure, the closer to the ray source, the wider the coverage of the ray; the closer to the detector array, the narrower the coverage of the ray. By designing the number and positions of ray sources, the wide width (i.e., the width at the first position) and the narrow width (i.e., the width at the second position) of ray coverage within the region of interest ROI are not less than the wide width and the narrow width of ray coverage in the single-source spiral CT mode shown in FIG. 10A .Therefore, in the CT scanning system provided by the embodiment of the present disclosure, under the condition of greatly saving detectors, a scanning speed not lower than that of the original single-source spiral CT may be obtained, and meanwhile, a scanning path formed by multiple sources is denser, which brings more advantages in the aspect of imaging quality.

In some exemplary embodiments of the present disclosure, the ray beams emitted by at least one of the m target points 10 is shaped as a fan-shaped beam. For example, the ray beams emitted by the m target points 10 may each be shaped as a fan-shaped beam. Therefore, for each target point of the distributed ray source, an inclined fan beam is formed relative to the detector, so that the scattered signal intensity is smaller than that of a cone beam formed by a single-source multi-row detector, and the radiation protection pressure of the CT scanning device is favorably reduced.

Based on this, some exemplary embodiments of the present disclosure provide a CT scanning system, and the system includes: a conveying device, configured to move a scanned object in a scanning channel in a predetermined conveying direction; a distributed ray source, where the distributed ray source includes m target points, the m target points are configured to be activated in a predetermined order to emit ray beams, m is a positive integer greater than or equal to 2, the ray beams emitted by at least one of the m target points is modulated into a first ray beam having a first energy, and the ray beams emitted by at least another of the m target points is modulated into a second ray beam having a second energy, where the first energy is lower than the second energy; a detector array, where the detector array is configured to detect a ray emitted from the distributed ray source and passing through the scanned object, and to generate projection data according to the detected ray, wherein the detector array is configured to generate first projection data in response to the first ray beam passing through the scanned object, and generate second projection data in response to the second ray beam passing through the scanned object; and an image reconstruction device, where the image reconstruction device is configured to generate a computed tomography image of the scanned object according to the first projection data and the second projection data. The distributed ray source is configured to rotate around a first axis when the scanned object is scanned, and the first axis is parallel to the conveying direction. In the distributed ray source, at least two of the m target points are offset in a tangent direction, and the tangent direction is a direction extending along a tangent line tangent to a rotation direction of the distributed ray source. In the CT scanning system, a distributed ray source technology and a rotary spiral CT scanning technology are combined into a whole, the distributed ray source is used for replacing a single-point ray source in the conventional spiral CT scanning system, and the CT scanning with high scanning speed may be implemented under the condition that the detector row number is not increased. Further, some target points of the distributed ray source are arranged in a staggered manner in the tangent direction of the rotation direction. Through such a staggered arrangement, the ray projection data contains lower information redundancy and higher information quality.

Still other exemplary embodiments of the present disclosure provide a CT scanning system, and the system includes: a conveying device, configured to move a scanned object in a scanning channel in a predetermined conveying direction; a distributed ray source, where the distributed ray source includes m target points, the m target points are configured to be activated in a predetermined order to emit ray beams, m is a positive integer greater than or equal to 2, the ray beams emitted by at least one of the m target points is modulated into a first ray beam having a first energy, and the ray beams emitted by at least another of the m target points is modulated into a second ray beam having a second energy, where the first energy is lower than the second energy; a detector array, where the detector array is configured to detect a ray emitted from the distributed ray source and passing through the scanned object, and to generate projection data according to the detected ray, wherein the detector array is configured to generate first projection data in response to the first ray beam passing through the scanned object, and generate second projection data in response to the second ray beam passing through the scanned object; and an image reconstruction device, where the image reconstruction device is configured to generate a computed tomography image of the scanned object according to the first projection data and the second projection data. The CT scanning system further includes a post collimator, and the post collimator is located on a side of the detector array facing the distributed ray source. In the CT scanning system, a distributed ray source technology and a rotary spiral CT scanning technology are combined into a whole, the distributed ray source is used for replacing a single-point ray source in the conventional spiral CT scanning system, and the CT scanning with high scanning speed may be implemented under the condition that the detector row number is not increased. Further, by providing the post collimator, the influence of scattered photons on the image quality may be suppressed. In addition, in this embodiment, the distributed ray source is applied to a spiral CT scanning system, and a single-row detector may be used, so that the structural complexity of the post collimator may be reduced, the processing difficulty of the post collimator may be reduced, and the cost of the post collimator may be reduced.

Still other exemplary embodiments of the present disclosure further provide a CT scanning system, and the system includes: a conveying device, configured to move a scanned object in a scanning channel in a predetermined conveying direction; a distributed ray source, where the distributed ray source includes m target points, the m target points are configured to be activated in a predetermined order to emit ray beams, m is a positive integer greater than or equal to 2, the ray beams emitted by at least one of the m target points is modulated into a first ray beam having a first energy, and the ray beams emitted by at least another of the m target points is modulated into a second ray beam having a second energy, where the first energy is lower than the second energy; a detector array, where the detector array is configured to detect a ray emitted from the distributed ray source and passing through the scanned object, and to generate projection data according to the detected ray, wherein the detector array is configured to generate first projection data in response to the first ray beam passing through the scanned object, and generate second projection data in response to the second ray beam passing through the scanned object; and an image reconstruction device, where the image reconstruction device is configured to generate a computed tomography image of the scanned object according to the first projection data and the second projection data. The detector array includes at least one row of detectors, each row of detectors includes a plurality of detector modules, and the detector modules of the at least one row of detectors are continuously arranged along an arc line in a plane perpendicular to the first axis; and at least one target point is offset from the arc center of the arc line in a plane perpendicular to the first axis. In the CT scanning system, a distributed ray source technology and a rotary spiral CT scanning technology are combined into a whole, the distributed ray source is used for replacing a single-point ray source in the conventional spiral CT scanning system, and the CT scanning with high scanning speed may be implemented under the condition that the detector row number is not increased. Further, the detector is arranged in a non-centripetal manner relative to the target point, which is beneficial to reducing the size of the CT scanning device and the weight of the device.

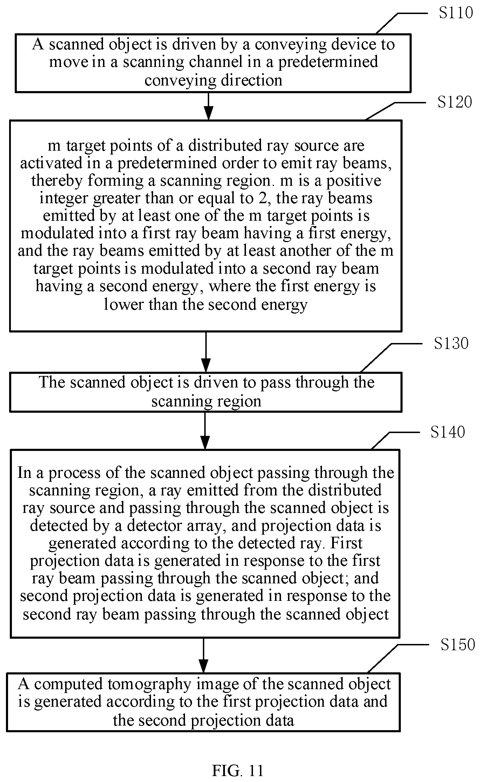

Embodiments of the present disclosure further provide a CT scanning method, and FIG. 11 is a flowchart of the CT scanning method according to some exemplary embodiments of the present disclosure, and as shown in FIG. 11, the CT scanning method may include steps S110 to S150. It should be noted that steps S110 to S150 are not limited to the order of the CT scanning method, and the CT scanning method may be executed in parallel or in a sequence different from the order described in the text in the case of no conflict.

In step S110, a scanned object 30 is driven by a conveying device 3 to move in a scanning channel 31 in a predetermined conveying direction Z.

In step S120, m target points 10 of a distributed ray source 1 are activated in a predetermined order to emit ray beams, thereby forming a scanning region. m is a positive integer greater than or equal to 2, the ray beams emitted by at least one of the m target points is modulated into a first ray beam having a first energy, and the ray beams emitted by at least another of the m target points is modulated into a second ray beam having a second energy, where the first energy is lower than the second energy.

In step S130, the scanned object 30 is driven to pass through the scanning region.

In step S140, in a process of the scanned object 30 passing through the scanning region, a ray emitted from the distributed ray source 1 and passing through the scanned object is detected by a detector array 2, and projection data is generated according to the detected ray. First projection data is generated in response to the first ray beam passing through the scanned object; and second projection data is generated in response to the second ray beam passing through the scanned object.

In step S150, a computed tomography image of the scanned object is generated according to the first projection data and the second projection data.

In an embodiment of the present disclosure, the distributed ray source 1 and the detector array 2 are configured to rotate about a first axis AX1 as the scanned object 30 is scanned, and the scanned object 30 moves through the scanning region as it is scanned.

In the distributed ray source 1, at least two target points of the m target points 10 are arranged offset in the tangential direction D2. Part of target points of the distributed ray source are arranged in a staggered mode in the tangential direction of the rotating direction, and through the staggered arrangement, the ray projection data contains less information redundancy and higher information quality.

For example, the distributed ray source 1 may have 5 target points that may independently emit beams, and the detector array 2 is a single-row detector.

The scanning method may be performed as follows. A CT scanning system is started, and the slip ring rotates. The scanned object enters the scanning channel via the entrance of the conveying device 3. The 5 source points of the ray source emit beams alternately according to the time sequence shown in FIG. 3C, and the detector and the digital acquisition system complete data acquisition for each output. The slip ring rotates for one circle, the ray source 1 finishes 360 rounds of beam outgoing, namely, the detector acquires 5 × 360 lines of projection data; the data processing system processes data; the scanned object 30 leaves the exit of the conveying device 3 and the scanning is completed.

The number of target points, the number of detector rows, the ray beam-emitting times of the ray source when the slip ring rotates for one circle and other geometric parameters in the CT scanning system may be adjusted according to different practical application requirements. The arrangement mode and the geometric parameters of the source and the detector are required to follow the requirement that the coverage of the X-ray to the to-be-reconstructed region is not repeated and omitted, and the data condition for implementing the spiral CT reconstruction is met.

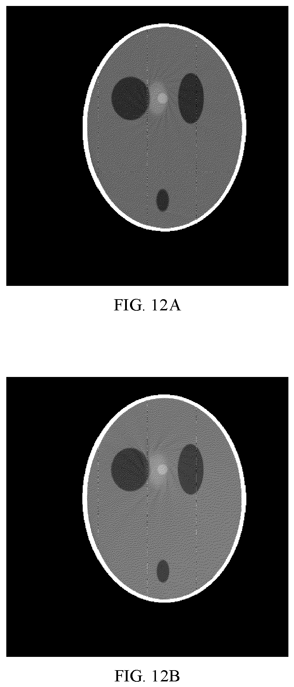

FIG. 12A and 12B illustrate a contrast image of a CT scanning system according to an embodiment of the present disclosure and a conventional spiral CT system under the same scanning parameters. As may be seen from FIG. 12A and 12B, a CT scanning at a high scanning speed may be achieved without increasing the number of detector rows, and the quality of a reconstructed image may be ensured at a high scanning speed.

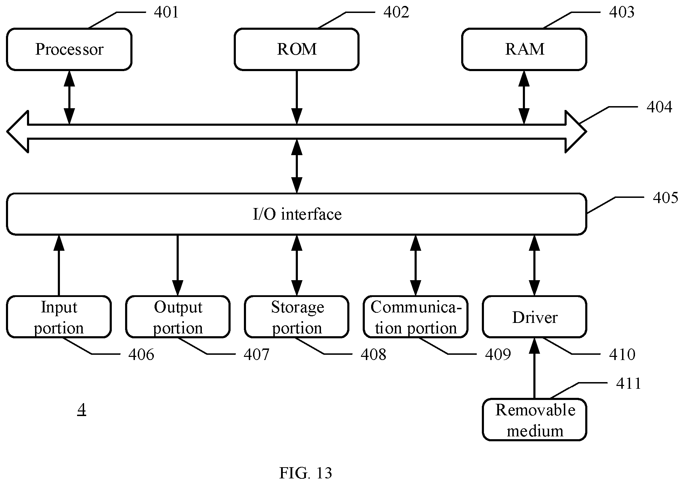

FIG. 13 schematically shows a block diagram of an imaging device of a CT scanning system according to an embodiment of the present disclosure.

As shown in FIG. 13, the imaging device 4 of the CT scanning system according to the embodiment of the present disclosure may include a processor 401, which may perform various appropriate actions and processes according to a program stored in a Read Only Memory (ROM) 402 or a program loaded from a storage section 408 into a Random Access Memory (RAM) 403. Processor 401 may include, for example, a general purpose microprocessor (e.g., a CPU), an instruction set processor and/or associated chipset, and/or a special purpose microprocessor (e.g., an Application Specific Integrated Circuit (ASIC)), among others. The processor 401 may also include on-board memory for caching purposes. Processor 401 may include a single processing unit or a plurality of processing units for performing the different actions of the method flows according to the embodiments of the present disclosure.

In the RAM 403, various programs and data necessary for the operation of the electronic device 400 are stored. The processor 401, ROM 402 and RAM 403 are connected to each other by a bus 404. The processor 401 performs various operations of the method flows according to the embodiments of the present disclosure by executing programs in the ROM 402 and/or the RAM 403. Note that the program may also be stored in one or more memories other than the ROM 402 and the RAM 403. The processor 401 may also perform various operations of method flows according to embodiments of the present disclosure by executing programs stored in the one or more memories.

Electronic device 400 may also include input/output (I/O) interface 405, input/output (I/O) interface 405 also connected to bus 404, according to an embodiment of the present disclosure. Electronic device 400 may also include one or more of the following components connected to I/O interface 405: an input portion 406 including a keyboard, a mouse, and the like; an output section 407 including a display device such as a Cathode Ray Tube (CRT), a Liquid Crystal Display (LCD), and the like, and a speaker; a storage section 408 including a hard disk and the like; and a communication section 409 including a network interface card such as a LAN card, a modem, or the like. The communication section 409 performs communication processing via a network such as the internet. A drive 410 is also connected to the I/O interface 405 as needed. A removable medium 411 such as a magnetic disk, an optical disk, a magnetooptical disk, a semiconductor memory, or the like is mounted on the drive 410 as needed, so that a computer program read out therefrom is mounted in the storage section 408 as needed.

The flowchart and block diagrams in the figures illustrate the architecture, functionality, and operation of possible implementations of systems, methods and computer program products according to various embodiments of the present disclosure. In this regard, each block in the flowchart or block diagrams may represent a module, segment, or portion of code, which includes one or more executable instructions for implementing the specified logical function(s). It should also be noted that, in some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams or flowchart illustration, and combinations of blocks in the block diagrams or flowchart illustration, may be implemented by special purpose hardware-based systems that perform the specified functions or acts, or combinations of special purpose hardware and computer instructions.

The embodiments of the present disclosure are described above. However, these examples are for illustrative purposes only and are not intended to limit the scope of the present disclosure. Although the embodiments are described separately above, this does not mean that the measures in the embodiments may not be used advantageously in combination. The scope of the disclosure is defined by the appended claims and equivalents thereof. Various alternatives and modifications may be devised by those skilled in the art without departing from the scope of the disclosure, and these alternatives and modifications are intended to fall within the scope of the disclosure.

## Claims

1. A CT scanning system, comprising:
a conveying device, configured to move a scanned object in a scanning channel in a predetermined conveying direction;
a distributed ray source, wherein the distributed ray source comprises m target points, the m target points are configured to be activated in a predetermined order to emit ray beams, m is a positive integer greater than or equal to 2, the ray beams emitted by at least one of the m target points is modulated into a first ray beam having a first energy, and the ray beams emitted by at least another of the m target points is modulated into a second ray beam having a second energy, wherein the first energy is lower than the second energy;
a detector array, wherein the detector array is configured to detect a ray emitted from the distributed ray source and passing through the scanned object, and to generate projection data according to the detected ray, wherein the detector array is configured to generate first projection data in response to the first ray beam passing through the scanned object, and generate second projection data in response to the second ray beam passing through the scanned object; and
an image reconstruction device, wherein the image reconstruction device is configured to generate a computed tomography image of the scanned object according to the first projection data and the second projection data,
wherein the distributed ray source is configured to rotate around a first axis when the scanned object is scanned, and the first axis is parallel to the conveying direction.

2. The system according to claim 1, wherein the system further comprises a voltage supply device, the voltage supply device is configured to respectively provide voltage to the m target points of the distributed ray source; and
the voltage supply device is configured to supply a first voltage to at least one of the m target points, and supply a second voltage to at least another of the m target points, wherein the first voltage is lower than the second voltage.

3. The system according to claim 1, wherein the system further comprises a voltage supply device, the voltage supply device is configured to respectively provide voltage to the m target points of the distributed ray source; and
the voltage supply device is configured to supply a first voltage to at least one of the m target points, and supply a second voltage to at least another of the m target points, wherein the first voltage is substantially equal to the second voltage.

4. The system according to claim 3, wherein the system further comprises at least one ray filter, the at least one ray filter is arranged on a beam path of at least one of the m target points, and is configured to modulate the ray beams emitted by at least one of the m target points into the first ray beam having the first energy.

5. The system according to any one of claims 1 to 4, wherein the system further comprises a controller, the controller is configured to control at least one aspect of the distributed ray source:
activation moment of the m target points, duration of the ray beams emitted by the m target points, intensity of the ray beams emitted by the m target points, and energy of the ray beams emitted by the m target points.

6. The system according to any one of claims 1 to 5, wherein in the distributed ray source, the m target points are arranged at intervals in a first arrangement direction, and the first arrangement direction is parallel to the first axis.

7. The system according to any one of claims 1 to 6, wherein the distributed ray source comprises an X-ray generating tube having a plurality of target points, or the distributed ray source comprises a plurality of X-ray generating tubes having a single target point.

8. The system according to any one of claims 1 to 7, wherein the detector array comprises a single row of detectors.

9. The system according to any one of claims 1 to 7, wherein the detector array comprises n rows of detectors, and n is a positive integer greater than or equal to 2.

10. The system according to any one of claims 1 to 7, wherein the detector array comprises an area array detector.

11. The system according to any one of claims 1 to 7, wherein the detector array includes n1 rows of detectors, n1 is a positive integer greater than or equal to 1, and m is greater than n1.

12. The system according to claim 9, wherein the m target points are arranged at intervals in a first arrangement direction, the first arrangement direction is parallel to the first axis; and the n rows of detectors are arranged at intervals in the first arrangement direction.

13. The system according to claim 1, wherein in the distributed ray source, at least two of the m target points are offset in a tangent direction, and the tangent direction is a direction extending along a tangent line tangent to a rotation direction of the distributed ray source.

14. The system according to any one of claims 1 to 13, wherein the CT scanning system further comprises a post collimator, and the post collimator is located on a side of the detector array facing the distributed ray source.

15. The system according to any one of claims 8, 9, 11, 12 and 14, wherein each row of detectors comprises a plurality of detector modules, and in a plane perpendicular to the first axis, a plurality of detector modules of at least one row of detectors are arranged continuously along an arc line; and
in a plane perpendicular to the first axis, at least one target point is offset from an arc center of the arc line.

16. A CT scanning method, comprising:
driving, by a conveying device, a scanned object to move in a scanning channel in a predetermined conveying direction;
activating m target points of a distributed ray source in a predetermined order to emit ray beams, to form a scanning region, wherein m is a positive integer greater than or equal to 2, the ray beams emitted by at least one of the m target points is modulated into a first ray beam having a first energy, and the ray beams emitted by at least another of the m target points is modulated into a second ray beam having a second energy, wherein the first energy is lower than the second energy;
driving the scanned object to pass through the scanning region;
in a process of the scanned object passing through the scanning region, detecting, by a detector array, a ray emitted from the distributed ray source and passing through the scanned object, and generating projection data according to the detected ray, wherein first projection data is generated in response to the first ray beam passing through the scanned object; and second projection data is generated in response to the second ray beam passing through the scanned object; and
generating a computed tomography image of the scanned object according to the first projection data and the second projection data,
wherein the distributed ray source is configured to rotate around a first axis when the scanned object is scanned, and the first axis is parallel to the conveying direction.
